# EUROPEAN PATENT APPLICATION

(11) **EP 4 431 609 A1**
(43) Date of publication of application: **18.09.2024**
(21) Application number: 23161904.0
(22) Date of filing: 14.03.2023
(51) Int. Cl.: C12P 7/52, C12N 9/04, C12N 9/10, C12N 9/88

(54) **METHOD FOR IMPROVING 2, 4 DIHYDROXYBUTYRIC ACID PRODUCTION AND YIELD**

(71) Applicant: Adisseo France S.A.S., 92160 Antony (FR); Institut National des Sciences Appliquées de Toulouse, 31000 Toulouse (FR); INSTITUT NATIONAL DE RECHERCHE POUR L'AGRICULTURE, L'ALIMENTATION ET L'ENVIRONNEMENT, 75007 Paris (FR); Centre national de la recherche scientifique, 75016 Paris (FR)
(72) Inventor: MALFOY, Thibault, 31520 RAMONVILLE-SAINT-AGNE (FR); BARTHE, Manon, 31000 TOULOUSE (FR); AURIOL, Clément, 31300 TOULOUSE (FR); FRANCOIS, Jean Marie, 31830 Plaisance du Touch (FR)
(74) Representative: Germain Maureau

(57) **Abstract**

The present invention relates to an improved method for the preparation of 2,4-dihydroxybutyrate from a carbon source *via* homoserine comprising a two steps pathway:
- a first step of conversion of the primary amino group of homoserine to a carbonyl group to obtain 2-oxo-4-hydroxybutyrate catalysed by an enzyme having homoserine transaminase activity as defined by E.C. 2.6.1.1 or E.C. 2.6.1.42 or E.C. 2.6.1.57 and
- a second step of reduction of the obtained 2-oxo-4-hydroxybutyrate (OHB) to 2,4-dihydroxybutyrate catalysed by an enzyme having OHB reductase activity, the enzyme having OHB reductase activity is lactate dehydrogenase as defined by E.C.1.1.1.27 or E.C.1.1.1.28, or malate dehydrogenase as defined by E.C.1.1.1.37, E.C.1.1.1.82 or E.C.1.1.1.299, and
wherein at least one enzyme having aldolase activity as defined by E.C.4.1.2.28 is inactivated to prevent OHB degradation.

## Description

### TECHNICAL FIELD

The present invention relates to an improvement of the prior art method for the preparation of 2,4-dihydroxybutyrate (2,4-DHB) from a carbon source *via* homoserine, comprising a two-step pathway, wherein a first step consists in the conversion of the primary amino group of homoserine to a carbonyl group to obtain 2-oxo-4-hydroxybutyrate (OHB), and the second step consists in the reduction of the obtained 2-oxo-4-hydroxybutyrate to yield 2,4-DHB. The improvement of this method consists in reducing, even eliminating, the diversion of OHB to optimise the pathway flux from the carbon source to the compound of interest, i.e. DHB. The DHB thus produced is notably used in the production/synthesis of 2-hydroxy-4-(methylthio)-butyrate (HMTB) and/or of the hydroxy-analogue of selenomethionine (HMSeBA or HMSeB) according to the present invention. The present invention also relates to a modified microorganism for the preparation of 2,4-DHB from a carbon source *via* homoserine comprising a two steps pathway wherein the pathway flux from the carbon source to the compound of interest, i.e. DHB, is optimised; and to a method of production of 2,4-DHB by culturing said modified microorganism.

### PRIOR ART

2,4-dihydroxybutyric acid (equally 2,4-DHB or DHB) is a compound of considerable economic interest. DHB can be readily converted into α-hydroxy-γ-butyrolactone in aqueous media by adjusting the appropriate pH. α-hydroxy-γ-butyrolactone is a prominent precursor for the production of the methionine substitute 2-hydroxy-4-(methylthio)-butyrate (HMTB) (US 2009/318715) or for the production of the hydroxy-analogue of selenomethionine (HMSeBA or HMSeB; EP1778706 B1), which have a large market in animal nutrition. At present, α-hydroxy-γ-butyrolactone may be prepared industrially by two different chemical methods:
*i*) derived from γ-butyrolactone by a multi-stage chemical process that implies halogenation of the γ-butyrolactone in position α, and subsequent substitution of the halogen atom by a hydroxyl group in alkaline medium (Deck *et al.,* 2008), and
*ii*) a two-steps chemical reaction from HMTB, *i.e.* alkylation followed by a hydrolysis (EP2627645 B1).

From growing oil prices, the need for the production of DHB from renewable resources arises. Microorganisms are capable of transforming biomass-derived raw material, *e.g.* sugars or organic acids, into a large variety of different chemical compounds (Werpy & Petersen, 2004). With the growing body of biochemical and genomic information, it is possible to modify microorganisms such that they overproduce naturally occurring metabolic intermediates with high yield and productivity (Bailey, 1991). Optimisation of production microorganisms often requires rational engineering of metabolic networks, which ensures, among others, overexpression of enzymes required for the biosynthesis of the metabolite of interest, and alleviation of product feedback inhibition. Another possibility is the implementation of novel enzymatic systems that catalyse the production of a non-natural metabolite of industrial relevance.

OHB, like DHB, is a non-natural product in microorganisms. Document WO 2014/009435 describes for the first time the conversion of homoserine to 2,4-DHB in a modified microorganism using a two-step enzymatic conversion including 2-oxo-4-hydroxybutyrate (OHB) reduction catalysed by an engineered malate dehydrogenase. However, this DHB production pathway in not optimised and the carbon flow, in particular OHB as an enzymatic substrate, can be diverted from the DHB production pathway to other competitive pathways, hence reducing the final yield of DHB. However, as OHB is a non-natural metabolite in microorganisms, the occurrence of these potential competitive, which is not obvious for the skilled person, must be identified and eventually removed. In other words, optimisation of the production yield of DHB, by the two-step enzymatic conversion via 2-oxo-4-hydroxybutyrate (OHB) as described in prior art, shall require:
- Optimising the pathway flux from carbon source to the compound of interest by overexpressing limiting enzymes of the pathway; and/or
- Removing unwanted reaction that loses carbon into by-products, such as organic acids or CO₂ as well as attenuate; and/or
- Removing the competitive pathway for a common intermediate between side pathway and production pathway.

Thus, it is critical to identify potential pathways that avoid loss of OHB and/or favouring its rerouting from DHB production. However, as above mentioned, since OHB is a non-natural product in the metabolism of microorganisms, one might expect that putative OHB consuming pathway may exist and or may be activated in response to the presence of this unusual metabolite, which have been neither proposed nor described in previous prior art, as regarding DHB production or other application. It is therefore not obvious to the skilled person how to ensure optimal DHB production.

As above mentioned, it was observed that the two-step pathway for the production of 2,4-DHB from homoserine (*i.e.* according to WO 2014/009435), notably comprises a coproduction of formaldehyde and formate with DHB. However, formaldehyde is widely known to be highly cytotoxic and mutagenic, whereas formate can pose problem in the downstream processing of DHB. Therefore, the prevention of the production of these co-products shall enhance the DHB production pathway, and by extension of HMTB and/or HMSeB production, in particular a HMTB and/or HMSeB chemical production (*i.e.* apart from the microorganism).

It is therefore necessary to reduce, or even delete, the diversion of the carbon flow in order to optimise the production of DHB and its by-products, such as HMTB and/or HMSeB, while suppressing the production of secondary products that are deleterious/toxic in particular for the producing microorganism. The present invention has as an objective to satisfy these needs.

### DESCRIPTION OF THE INVENTION

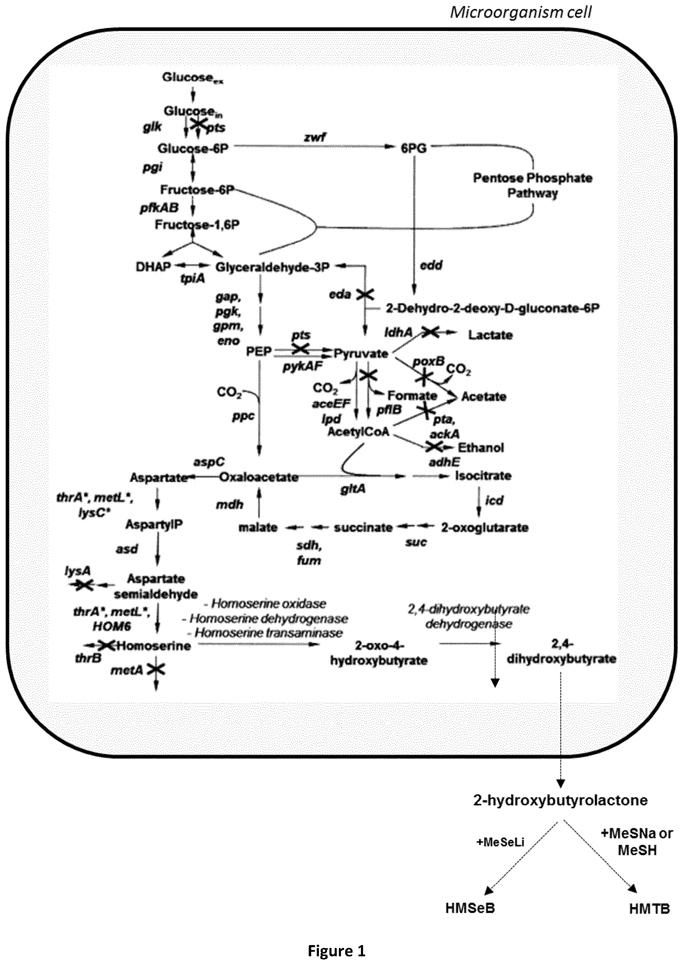

Accordingly, one object of the present invention is an improved method for the preparation of 2,4-DHB from a carbon source *via* homoserine, in particular from homoserine, comprising a two-step pathway (see Figure 1):
- a first step of conversion of the primary amino group of homoserine to a carbonyl group to obtain OHB, and
- a second step of reduction of the obtained OHB to 2,4-DHB,
wherein the pathway flux from carbon source to the compound of interest is optimised, in particular OHB, as an enzymatic substrate, is not diverted from the DHB production pathway, and by extension of HMTB and/or HMSeB production, in particular a HMTB and/or HMSeB chemical production (i.e. apart from the microorganism).

The inventors unexpectedly and surprisingly observed that the inactivation, deletion or alteration of the enzymatic activities of the so called aldolase enzymes or preferentially pyruvate aldolase class I and class II enzymes, with E.C 4.1.2.28, resulted in a higher DHB production, and by extension a higher HMTB and/or HMSeB production, in particular a chemical production method (*i.e.* apart from the microorganism) than described in prior art, in particular by document WO 2014/009435 or the production obtained by implementing the microorganism described in WO 2014/009435. Besides, this inactivation, deletion or alteration of the enzymatic activities of aldolase enzymes, with E.C 4.1.2.28, reduces and/or prevents the production of toxic by-products such as formaldehyde.

Thus, the present invention concerns a method for the preparation of 2,4-dihydroxybutyrate and/or, by extension, of HMTB and/or HMSeB, in particular a chemical production method of HMTB and/or HMSeB, from a carbon source *via* homoserine, in particular from homoserine, comprising a two steps pathway:
- a first step of conversion of the primary amino group of homoserine to a carbonyl group to obtain 2-oxo-4-hydroxybutyrate catalysed by an enzyme having homoserine transaminase activity as defined by E.C. 2.6.1.1 or E.C. 2.6.1.42 or E.C. 2.6.1.57 and
- a second step of reduction of the obtained 2-oxo-4-hydroxybutyrate (OHB) to 2,4-dihydroxybutyrate catalysed by an enzyme having OHB reductase activity, the enzyme having OHB reductase activity is lactate dehydrogenase as defined by E.C.1.1.1.27 or E.C.1.1.1.28, or malate dehydrogenase as defined by E.C.1.1.1.37, E.C.1.1.1.82 or E.C.1.1.1.299, and wherein at least one enzyme having aldolase activity as defined by E.C.4.1.2.28 is inactivated to prevent OHB degradation.

Indeed, Bouzon reported the production of OHB by condensation of pyruvate and formaldehyde, using pyruvate dependent aldolase (Bouzon *et al.,* 2017). While it was reported that several promiscuous aldolase was able to catalyse the condensation of pyruvate with formaldehyde into OHB as an non-natural intermediates of synthetic pathway enabling assimilation of CO2 by bacteria (Bouzon *et al*., 2017), it was not obvious that the loss of these promiscuous enzyme would enhance DHB production.

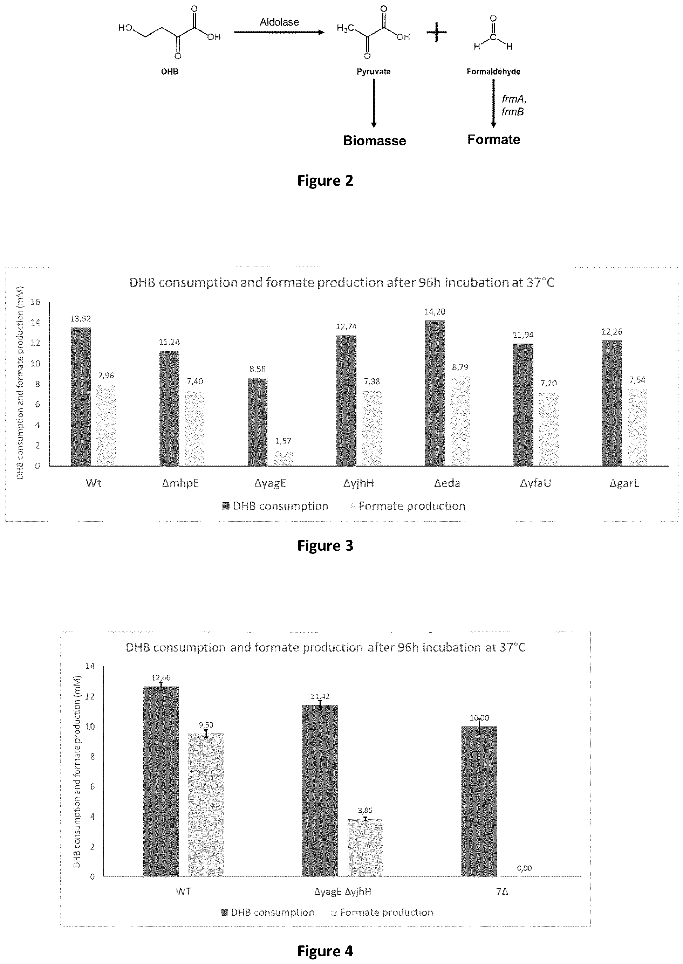

A method for production of 2,4-DHB and/or, by extension, of HMTB and/or HMSeB, in particular a chemical production method of HMTB and/or HMSeB, according to the invention provides an improved/optimised preparation of 2,4-dihydroxybutyrate, by extension of HMTB and/or HMSeB, wherein the degradation of OHB notably into formaldehyde and then formate, is prevented (See Figure 2). It is thus responsible for significant increases in yield, in volumetric productivity, and in DHB concentration and/or, by extension, of HMTB and/or HMSeB concentration.

The inactivation of at least one enzyme having aldolase activity as defined by E.C.4.1.2.28 therefore allows a significant gain in initial substrate yield (*e.g.* glucose), and also avoids the accumulation of formate, an organic acid that can potentially hinder the purification of 2,4-DHB and/or, by extension, the production of HMTB and/or the production of HMSeB, in particular a chemical production method of HMTB and/or HMSeB.

This invention therefore allows significant gains in carbon yield, and therefore in the cost of any biotechnological process operating with the method and/or modified microorganism according to the invention.

The carboxylic acids cited within the present application are equally named under their salt (*e.g.* 2,4-dihydroxyburyrate) or acid forms (*e.g.* 2,4-dihydroxybutyric acid).

In the description, enzymatic activities are also designated by reference to the genes coding for the enzymes having such activity. The use of the denomination of the genes is not limited to a specific organism, but covers all the corresponding genes and proteins in other organisms (*e.g.* microorganisms, functional analogues, functional variants and functional fragments thereof as long as they retain the enzymatic activity).

In the context of the invention, the expression "genetically modified microorganism" means that the microorganism according to the invention is not found in nature and is modified by introduction of new genetic elements and/or by deletion and/or modification of the endogenous genetic elements of the microorganism. This modification may be generated by genome editing, *in vivo* evolution, genomic recombination, directed or random mutagenesis or any other strain engineering technique known to the skilled person.

In the context of the invention, the terms "inactivation" and "alteration" and "deletion" are used interchangeably and refer to the absence of production of the corresponding protein or the production of a non-functional corresponding protein obtained, for example, by the implementation of the gene silencing technique corresponding to transcriptional gene silencing or post-transcriptional gene silencing, by the introduction of a mutation in the promoter sequence which controls the expression of the gene coding for said protein and/or in the coding sequence for said protein, by the shift of the reading frame causing the introduction of an early stop codon in the coding sequence for the protein or by the deletion of the gene coding for the promoter which controls the expression of the gene coding for said protein and/or of the gene coding for said protein.

In the context of the invention, the terms "improved", "optimised", "enhanced" and "increased" are used interchangeably and refer to a higher activity including enzymatic activity and/or a higher production of a compound compared to the same "non-improved" microorganism.

In the context of the invention, the terms "identical" and "homologous" are used interchangeably and refer to the sequence identity between two polypeptides. When a position in each of the two sequences being compared is occupied by the same monomeric amino acid subunit, then the molecules are homologous or identical at that position. The percentage identity between two sequences is a function of the number of corresponding positions shared by the two sequences, and is this number divided by the number of positions compared and multiplied by 100. For example, if 6 out of 10 positions in two matched sequences are identical, then the two sequences are 60% identical. As a general rule, the comparison is performed by aligning the two sequences to give maximum homology/identity. Various bioinformatics tools known to the one skilled in the art might be used to align nucleic acid sequences such as BLAST or FASTA.

The terms "coding" or "coding for", "code" or "code for" are used interchangeably and refer to the inherent property of specific nucleotide sequences in a polynucleotide, such as a gene, cDNA or mRNA, to serve as a template for the synthesis of other polymers with a defined sequence of amino acids, and the biological properties that result from this. Thus, a gene codes for a protein if transcription and translation of the mRNA corresponding to that gene produces the protein in a cell or other biological system. Both the coding strand, whose nucleotide sequence is identical to the mRNA sequence, and which is generally described in sequence listings and databases, and the non-coding strand, which is used as a template for transcription of a gene or cDNA, may be designated as coding for the protein or other product of that gene or cDNA. Thus, in the context of the present invention, the expression "substantial homology" covers homology with respect to structure and/or amino acid components and/or biological activity. Methods for performing sequence alignment and determining sequence identity are known to the skilled artisan, may be performed without undue experimentation, and calculations of identity values may be obtained with definiteness. See, for example, Ausubel, *et al.,* eds. (1995); and the ALIGN program (Dayhoff (1978). A number of algorithms are available for aligning sequences and determining sequence identity and include, for example, the homology alignment algorithm of Needleman *et al.,* (1970); the local homology algorithm of Smith, *et al.,* (1981); the search for similarity method of Pearson, *et al.,* (1988); the Smith-Waterman algorithm (Shpaer (1997)); and BLASTP, BLASTN, and BLASTX algorithms (see Altschul, *et al.,* (1990)). Computerized programs using these algorithms are also available, and include, but are not limited to: ALIGN or Megalign (DNASTAR) software, or WU-BLAST-2 (Altschul, *et al.,* (1996)); or GAP, BESTFIT, BLAST (Altschul, *et al.* (1997)), supra, FASTA, and TFASTA, available in the Genetics Computing Group (GCG) package, Version 8, Madison, Wis., USA; and CLUSTAL in the PC/Gene program by Intelligenetics, Mountain View, Calif. Those skilled in the art can determine appropriate parameters for measuring alignment, including algorithms needed to achieve maximal alignment over the length of the sequences being compared. Preferably, the sequence identity is determined using the default parameters determined by the program. Specifically, sequence identity can be determined by the Smith-Waterman homology search algorithm (Shpaer, 1997) as implemented in MSPRCH program (Oxford Molecular) using an affine gap search with the following search parameters: gap open penalty of 12, and gap extension penalty of 1. Preferably, paired amino acid comparisons can be carried out using the GAP program of the GCG sequence analysis software package of Genetics Computer Group, Inc., Madison, Wis., employing the blosum 62 amino acid substitution matrix, with a gap weight of 12 and a length weight of 2. With respect to optimal alignment of two amino acid sequences, the contiguous segment of the variant amino acid sequence may have additional amino acid residues or deleted amino acid residues with respect to the reference amino acid sequence. The contiguous segment used for comparison to the reference amino acid sequence will include at least 20 contiguous amino acid residues, and may be 30, 40, 50, or more amino acid residues. Corrections for increased sequence identity associated with inclusion of gaps in the derivative's amino acid sequence can be made by assigning gap penalties.

In the context of the present invention, the expression "carbon source" refers to a nutrient providing the carbon necessary for the production of enzymes according to the invention. This so-called "carbon source" is a small molecule with a low molecular weight, *i.e.* a molecular weight of 500 g/mol or less, such as a monosaccharide, a diholoside or a polyol (e.g. glucose).

In the context of the present invention, the expression "*via* homoserine" means that homoserine is an intermediate substrate in the DHB production pathway and/or, by extension, in the HMTB and/or HMSeB chemical production method.

In the context of the present invention, any amino acid substitutions are "conservative amino acid substitutions" using L-amino acids, wherein one amino acid is replaced by another biologically similar amino acid. Conservative amino acid substitutions are those that preserve the general charge, hydrophobicity/hydrophilicity, and/or steric bulk of the amino acid being substituted. Examples of conservative substitutions are those between the following groups: Gly/Ala, Val/Ile/Leu, Lys/Arg, Asn/Gln, Glu/Asp, Ser/Cys/Thr, and Phe/Trp/Tyr. A derivative may, for example, differ by as few as 1 to 10 amino acid residues, such as 6-10, as few as 5, as few as 4, 3, 2, or even 1 amino acid residue.

In the context of the present invention, the expression "functional variant" encompasses enzymes that may present substantial sequence modifications when compared to the sequences specifically described within the present application but that still retain the original enzymatic activity. It also means that the sequence of the enzyme may comprise less amino acids than the original one but said truncated enzyme still retains the original enzymatic activity.

In the context of the present invention, the expression "improve the activity and/or substrate affinity" means that the enzyme before mutation, was either unable to use the substrate, and/or synthesized the product of the reaction at a maximum specific rate at least three times lower, and/or had an affinity for homoserine or OHB that was at least three times lower, and/or had a maximum specific activity on the natural substrate that was at least three times higher, and/or had an affinity for the natural substrate that was at least three times higher.

In the context of the present invention, a "nucleic acid sequence" refers to a DNA or RNA molecule in single or double stranded form, preferably a DNA molecule. An "isolated DNA", as used herein, refers to a DNA which is not naturally-occurring or no longer in the natural environment wherein it was originally present, *e.g.,* a DNA coding sequence associated with other regulatory elements in a chimeric gene, a DNA transferred into another host cell, or an artificial, synthetically-made DNA sequence having a different nucleotide sequence compared to any naturally-occurring DNA sequence.

In the context of the present invention, the term "microorganism" is intended to mean any lower unicellular organism into which the chimeric gene(s), nucleic acid(s) or vector(s) according to the invention may be introduced in order to produce 2,4-DHB.

In the context of the present invention, the expression "functionally linked to one another" means that the elements of a chimeric gene are linked to one another in such a way that the function of one of these elements is affected by that of another. By way of example, a promoter is functionally linked to a coding sequence when it is capable of affecting the expression of said coding sequence. The construction of the chimeric gene according to the invention and the assembly of its various elements can be carried out using techniques well known to those skilled in the art. The choice of the regulatory elements constituting the chimeric gene depends essentially on the host organism in which they must function, and those skilled in the art are capable of selecting regulatory elements which are functional in a given host organism.

In the context of the present invention, the term "functional" is intended to mean capable of functioning in a given host organism.

Preferably, the present invention concerns a method for the preparation of 2,4-dihydroxybutyrate from a carbon source *via* homoserine, in particular from homoserine, comprising a two steps pathway, wherein at least one enzyme having aldolase activity as defined by E.C.4.1.2.28 is inactivated to prevent OHB degradation, as defined having the following features, taken alone or in combination:
- said enzyme implemented in first step is catalysed by an enzyme encoded by an endogenous or a heterologous gene;
- said enzyme implemented in the second step is catalysed by an enzyme encoded by an endogenous or a heterologous gene;
- said enzyme having aldolase activity is catalysed by an enzyme encoded by an endogenous or a heterologous gene;
- the enzyme having aldolase activity is encoded by sequence set forth in SEQ ID NO: 1 or SEQ ID NO: 3, or having a sequence as set forth in SEQ ID NO: 2 or SEQ ID NO: 4; or any sequence sharing a homology of at least 50%, advantageously at least 60%, at least 70% or even at least 80%, preferably at least 90%, at least 95%, at least 96%, at least 97%, at least 98% or even at least 99%, with said sequences SEQ ID NO: 1, 3, 2 or 4;
- the enzyme converting the primary amino group of homoserine to a carbonyl group to obtain OHB can be homoserine transaminase, homoserine dehydrogenase, or homoserine oxidase;
- the enzyme having homoserine transaminase activity can be identified among enzymes having aspartate transaminase (EC2.6.1.1) activity, branched-chain-amino-acid transaminase (EC2.6.1.42) activity, or aromatic-amino-acid transaminase (EC2.6.1.57) activity
- the carbon source is selected from the group consisting of a monosaccharide, a diholoside, a polyol and mixtures thereof;
- the carbon source is selected from the group consisting of glucose, sucrose, lactose, glycerol and mixtures thereof;
- the carbone source is glucose;
- the enzyme having homoserine transaminase activity is encoded by gene *ilvE, tyrB, aspC, araT, bcaT, alaC, AROB*; preferably the enzyme having homoserine transaminase activity can be the branched-chain-amino-acid transaminase from *Escherichia coli,* Ec-IIvE, and *Lactococcus lactis,* LI-BcaT, the homoserine transaminase activity can be the alanine-α-ketoglutarate transaminase from *E coli* Ec-AlaC, and more preferably the variant Ec-AlaCA142PY275D, also the aromatic-amino-acid transaminases from *E. coli,* Ec-TyrB, *L. lactis,* LI-AraT, and *Saccharomyces cerevisiae,* Sc-Aro8, or the aspartate transaminase from *E. coli,* Ec-AspC; or homologues thereof;
- the enzyme having homoserine transaminase activity is encoded by sequence set forth in SEQ ID NO: 5, SEQ ID NO: 7, SEQ ID NO: 9, SEQ ID NO: 11, SEQ ID NO: 13, SEQ ID NO: 15; SEQ ID NO: 126, SEQ ID NO: 128 or corresponds to SEQ ID NO: 6, SEQ ID NO: 8, SEQ ID NO: 10, SEQ ID NO: 12, SEQ ID NO: 14, SEQ ID NO: 16, SEQ ID NO: 127, SEQ ID NO: 129; or any sequence sharing a homology of at least 50%, advantageously at least 60%, at least 70% or even at least 80%, preferably at least 90%, at least 95%, at least 96%, at least 97%, at least 98% or even at least 99%, with said sequences SEQ ID NO: 5 to 16;
- the enzyme having OHB reductase is (D)-lactate dehydrogenase, preferably from *Escherichia coli*; (L)-lactate dehydrogenase, preferably from *Lactococcus lactis,* from *Oryetalagus cuniculus,* from *Geobacillus stearothermophilus,* or from *Bacillus subtilis*; (L)-malate dehydrogenase, preferably from *Escherichia coli*; or homologues thereof;
- the enzyme having OHB reductase is a lactate dehydrogenase comprising at least one mutation in position V17, Q85, E89, 1226 or A222 said positions being defined by reference to the *L-Lactis tA* (SEQ ID NO: 17 or SEQ ID NO: 18); and/or a malate dehydrogenase comprising at least one mutation in position I12, R81, M85, D86, V93, G179, T211 or M227 said positions being defined by reference to the *E.coli* Mdh (SEQ ID NO: 19 or SEQ ID NO: 20), wherein the naturally occurring amino acid in said positions is replaced by anyone of the other 19 naturally existing proteinogenic amino acids, that is by either alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine.;
- the enzyme having OHB reductase is a lactate dehydrogenase comprising at least one mutation in position V17, Q85N or I226V said positions being defined by reference to the L-*Lactis tA* (SEQ ID NO: 17 or SEQ ID NO: 18); and/or a malate dehydrogenase comprising at least one mutation in position I12V, R81A, M85Q, D86S, or G179D said positions being defined by reference to the *E.coli* Mdh (SEQ ID NO: 19 or SEQ ID NO: 20);
- the enzyme having OHB reductase is a lactate dehydrogenase encoded by sequence set in forth in SEQ ID NO: 21, SEQ ID NO: 23 or SEQ ID NO: 25 or corresponds to SEQ ID NO: 22, SEQ ID NO: 24, SEQ ID NO: 26; or any sequence sharing a homology of at least 50%, advantageously at least 60%, at least 70% or even at least 80%, preferably at least 90%, at least 95%, at least 96%, at least 97%, at least 98% or even at least 99%, with said sequences SEQ ID NO: 21 to 26;
- the enzyme having OHB reductase is a malate dehydrogenase encoded by sequence set in forth in SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43 or corresponds to SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44; or any sequence sharing a homology of at least 50%, advantageously at least 60%, at least 70% or even at least 80%, preferably at least 90%, at least 95%, at least 96%, at least 97%, at least 98% or even at least 99%, with said sequences SEQ ID NO: 27 to 44;
- the enzyme having OHB reductase activity is encoded by sequence set in forth in SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79 or SEQ ID NO: 81; or corresponds to SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80 or SEQ ID NO: 82; or any sequence sharing a homology of at least 50% , advantageously at least 60%, at least 70% or even at least 80%, preferably at least 90%, at least 95%, at least 96%, at least 97%, at least 98% or even at least 99%, with said sequences SEQ ID NO: 45 to 82;
- the enzymes according to the present invention having the same activity (either OHB reductase), or the enzyme converting the primary amino group of homoserine to a carbonyl group to obtain OHB or the enzyme having aldolase activity share at least about 50%, 70% or 85% amino acid sequence identity, preferably at least about 85% amino acid sequence identity, more preferably at least about 90% amino acid sequence identity, even more preferably at least about 95% amino acid sequence identity and yet more preferably 98%, or even 99% amino acid sequence identity;
- the activity of the enzyme catalysing the first and/or the second step of the method of the present invention is enhanced. This enhancement can be measured by an enzymatic assay as described in Examples. Improvement of said enzymes can be obtained by at least one mutation, said mutation(s) *i*) improving the activity and/or substrate affinity of the mutated enzyme for homoserine or OHB respectively, and or ii) decreasing the activity and/or substrate affinity of the mutated enzyme for their natural substrate.

The present invention also concerns a method for the preparation of hydroxy-methionine (HMTB) and/or hydroxy-selenomethionine (HMSeB) from a carbon source *via* homoserine, in particular from homoserine, comprising a two steps pathway, wherein at least one enzyme having aldolase activity as defined by E.C.4.1.2.28 is inactivated to prevent OHB degradation, as above defined. Indeed, 2,4-DHB is transformed into HMTB or HMSeB by cyclisation following by sodium mercaptan or lithium methaneselenolate addition, respectively and as described by documents EP 2 054 382 and EP 1 778 706.

The present invention also relates to a chimeric gene comprising, functionally linked to one another, at least one promoter, which is functional in a host organism, a polynucleotide encoding anyone of the enzymes catalysing first and second step of the method as defined according to the invention, and a terminator element that is functional in the same host organism. The various elements which a chimeric gene may contain are, firstly, elements regulating transcription, translation and maturation of proteins, such as a promoter, a sequence encoding a signal peptide or a transit peptide, or a terminator element constituting a polyadenylation signal and, secondly, a polynucleotide encoding a protein.

The promoters, which the chimeric gene according to the invention may contain, are either constitutive or inducible. By way of example, the promoters used for expression in bacteria may be chosen from the promoters mentioned below. For expression in *Escherichia coli* mention may be made of the *lac, trp, lpp, phoA, recA, araBAD, prou, cst-I, tetA, cadA, nar, tac, trc, Ipp-lac, Psyn, cspA, PL, PL-9G-50, PR-PL, 77, [lambda]PL-PT7, T3-lac, T5-lac, T4 gene 32, nprM-lac, VHb* and the *protein A* promoters or else the *Ptrp* promoter (WO 99/64607). For expression in Gram-positive bacteria such as *Corynebacteria* or *Streptomyces,* mention may be made of the *PtipA* or *PS1* and *PS2* (FR91/09870) promoters or those described in application EP0629699A2. For expression in yeasts and fungi, mention may be made of the *K. lactis PLAC4* promoters or the *K. lactis Ppgk* promoter (FR 91/05294), the *Trichoderma reesei tef1* or *cbh1* promoter (WO 94/04673), the *Penicillium funiculosum* his, *csl* or *apf* promoter (WO 00/68401) and the *Aspergillus niger gla* promoter.

According to the invention, the chimeric gene may also comprise other regulatory sequences, which are located between the promoter and the coding sequence, such as transcription activators (enhancers).

As such, the chimeric gene of the invention comprises in a specific embodiment at least, in the direction of transcription, functionally linked, a promoter regulatory sequence which is functional in a host organism, a nucleic acid sequence encoding a polynucleotide encoding anyone of the enzymes catalysing first and second step of the method as defined according to the invention and a terminator regulatory sequence which is functional in said host organism.

The present invention also relates to a cloning and/or expression vector comprising a chimeric gene according to the invention or a nucleic acid sequence of the invention. The vector according to the invention is of use for transforming a host organism and expressing in this organism anyone of the enzymes catalysing the first and/or the second step(s) of the method of the present invention. This vector may be a plasmid, a cosmid, a bacteriophage or a virus. Preferentially, the transformation vector according to the invention is a plasmid. Generally, the main qualities of this vector should be able to maintain itself and to self-replicate in the cells of the host organism, in particular by virtue of the presence of an origin of replication, and to express anyone of the enzymes catalysing the first and/or the second step(s) of the method of the present invention therein. For the purpose of stable transformation of a host organism, the vector may also integrate into the genome. The choice of such a vector, also the techniques of insertion of the chimeric gene according to the invention into this vector are part of the general knowledge of those skilled in the art. Advantageously, the vector used in the present invention also contains, in addition to the chimeric gene according to the invention, a chimeric gene encoding a selectable marker. This selectable marker makes it possible to select the host organisms that are effectively transformed, *i.e.* those that incorporated the vector. According to a particular embodiment of the invention, the host organism to be transformed is a bacterium, a yeast, a fungus. Among the selectable markers that can be used, mention may be made of markers containing genes for resistance to antibiotics, such as, for example, the hygromycinphosphotransferase gene. Other markers may be genes to complement an auxotrophy, such as the *pyrA, pyrB, pyrG, pyr4, arg4, argB* and *trpC* genes, the *molybdopterin synthase* gene or that of *acetamidase.* Mention may also be made of genes encoding readily identifiable enzymes such as the GUS enzyme, or genes encoding pigments or enzymes regulating the production of pigments in the transformed cells. in particular, such selectable marker genes are described in patent applications WO 91/02071, WO 95/06128, WO 96/38567 and WO 97/04103.

The improved/optimised DHB and/or, and by extension of HMTB and/or HMSeB production, in particular a chemical production method of HMTB and/or HMSeB, according to the invention can be implemented in a wild-type microorganism (*i.e.* non-genetically modified microorganism) or in a genetically modified microorganism, which have been already genetically modified, *i.e.* genetic modifications different from those described according to the present invention. Such genetic modifications may cause an increase in the production/concentration of an intermediate product involved in the production of DHB and/or, by extension HMTB and/or HMSeB chemical production. For example, it may be a microorganism genetically modified to produce homoserine in a higher content/yield than that produced by the wild-type microorganism.

More specifically, the modified microorganism for the preparation of 2,4-DHB and/or, by extension of HMTB and/or HMSeB, from a carbon source *via* homoserine, in particular from homoserine, comprising a two steps pathway comprising :
- a first step of conversion of the primary amino group of homoserine to a carbonyl group to obtain 2-oxo-4-hydroxybutyrate catalysed by an enzyme having homoserine transaminase activity as defined by E.C. 2.6.1.1, E.C. 2.6.1.42 or E.C. 2.6.1.57, and
- a second step of reduction of the obtained 2-oxo-4-hydroxybutyrate to obtain 2,4-dihydroxybutyrate catalysed by an enzyme having OHB reductase activity, the enzyme having OHB reductase activity is lactate dehydrogenase as defined by E.C.1.1.1.27 or E.C.1.1.1.28, or malate dehydrogenase as defined by E.C.1.1.1.37, E.C.1.1.1.82 or E.C.1.1.1.299,
wherein at least one enzyme having aldolase activity as defined by E.C.4.1.2.28 is inactivated to prevent OHB degradation.

Preferably, the present invention concerns a modified microorganism for an improved/optimised production of DHB and/or, by extension, HMTB and/or HMSeB production (in particular a chemical production method of HMTB and/or HMSeB) from a carbon source *via* homoserine comprising a two steps pathway, wherein at least one enzyme having aldolase activity as defined by E.C.4.1.2.28 is inactivated to prevent OHB degradation, as defined having the following features, taken alone or in combination:
- the enzymes involved in the two steps are those above described ;
- the microorganism is a fungus, for example of the *Penicillium, Aspergillus* and more particularly *Aspergillusflavus, Chrysosporium* or *Trichoderma* genus;
- the microorganism is a yeast, in particular of the *Saccharomycetaceae, Pichiaceae* or *Schizosaccharomycetaceae,* most preferentially *Saccharomyces cerevisiae, Schizosaccharomyces pombe, Kluyveromyces lactis, Kluyveromyces marxianus,* or *Pichia jadinii, Pichia stipitis* or *Pichia pastoris*;
- the microorganism is a bacterium, preferentially selected among *Enterobacteriaceae, Clostridiaceae, Bacillaceae, Streptomycetaceae, Si-repi-ococcaceae, Methylobacteriacae,* and *Corynebacteriaceae,* most preferentially *Escherichia coli, Bacillus subtilis, Corynebacterium glutamicum, Clostridium acetobutylicum, Methylobacterium extorquens* or *Lactococcus lactis.*

According to a specific embodiment, the present invention also relates to modified microorganisms containing at least one chimeric gene according to the invention, either integrated into their genome or carried on an extra-chromosomal genetic element, for example a plasmid. In a more specific aspect of the invention, the transformed host organism comprises a nucleic acid of the invention encoding a polypeptide converting the primary amino acid group of homoserine to a carbonyl group to obtain OHB and/or a nucleic acid encoding a polypeptide reducing OHB in 2,4-DHB or a chimeric gene comprising a nucleic acid encoding a polypeptide converting the primary amino acid group of homoserine to a carbonyl group to obtain OHB, and/or a OHB reductase or an expression vector comprising a nucleic acid encoding a polypeptide converting the primary amino acid group of homoserine to a carbonyl group to obtain OHB, or a polypeptide having a OHB reductase activity.

Within a further aspect of the invention, the synthetic pathway for the conversion of homoserine into DHB and/or, by extension, of HMTB and/or HMSeB production, in particular a HMTB and/or HMSeB chemical production method, is expressed in a microorganism with enhanced production of homoserine, *i.e.* with a higher homoserine production than the parent organism or a wild-type organism. Enhanced production of homoserine in microorganisms can be achieved by *i*) overexpressing the enzymes aspartate kinase, aspartate semialdehyde dehydrogenase, and homoserine dehydrogenase, *ii*) by rendering the aspartate kinase enzyme insensitive to product inhibition that can be brought about by lysine, methionine, or threonine, and *iii*) by deletion of metabolic pathways that branch off the homoserine biosynthesis pathway. Overexpression of aspartate kinase, aspartate semialdehyde dehydrogenase, and homoserine dehydrogenase can be achieved by expressing the enzymes from a multicopy plasmid under the control of an appropriate constitutive or inducible promoter. Alternatively, overexpression of said enzymes can be achieved by deletion of transcriptional repressors that limit the transcription of genes coding for aspartate kinase, aspartate semialdehyde dehydrogenase, and homoserine dehydrogenase. Aspartate kinases can be rendered insensitive to inhibition by aspartate-derived amino acids by introducing appropriate mutations into their amino acid sequences. Entry points into metabolic pathways that branch off the homoserine biosynthesis pathway are catalysed by enzymes having O-succinyl homoserine or O-acetyl homoserine synthase activity (entry into methionine biosynthesis), homoserine kinase activity (entry into threonine biosynthesis), or diaminopimelate decarboxylase activity (entry into lysine biosynthesis). Deletion of genes encoding proteins having said enzymatic activities avoids formation aspartate-derived amino acids and therefore aids homoserine formation (Figure 1).

Accordingly, deletion of the genes *metA, thrB,* and *lysA* in *E. coli* attenuates pathways that branch of the homoserine biosynthetic pathway. The increase of enzymatic activities of the homoserine pathway in *E. coli* can be achieved, for instance, by the overexpression of the bifunctional aspartate kinase-homoserine dehydrogenase mutant *thrA* S345F (insensitive to threonine inhibition) and asd (both genes from *E. coli*); or by the overexpression of the monofunctional aspartate kinase mutant *lysC* E250K (insensitive to lysine), asd (both genes from *E. coli*), and the homoserine dehydrogenase gene *HOM6* from *S*. *cerevisiae.*

The microorganism of the invention may also have attenuated capacity to export homoserine which increases the intracellular availability of this amino acid. In order to achieve decreased homoserine export from the cells, permeases capable of exporting homoserine can be deleted. Such permeases may be identified by overexpressing genomic libraries in the microorganism and cultivating said microorganism at inhibitory concentrations of homoserine or structurally similar amino acids such as threonine, leucine, or aspartate (Zakataeva *et al.,* (1999). Genes whose overexpression confers growth at increased concentrations of either of said amino acids are likely to participate in homoserine export.

In a further aspect, the microorganism of the invention being *E. coli* carries deletions in the homoserine efflux transporters *rhtA, rhtB,* and/or *rhtC.*

Accordingly, the microorganism of the invention may also exhibit enzymatic activities chosen among phosphoenolpyruvate carboxylase, phosphoenolpyruvate carboxykinase, isocitrate lyase, pyruvate carboxylase, and hexose symporter permease which is increased, and/or at least one of the enzymatic activities chosen among lactate dehydrogenase, alcohol dehydrogenase, acetate kinase, phosphate acetyltransferase, pyruvate oxidase, isocitrate lyase, fumarase, 2-oxoglutarate dehydrogenase, pyruvate kinase, malic enzyme, phosphoglucose isomerase, phosphoenolpyruvate carboxylase, phosphoenolpyruvate carboxykinase, pyruvate-formate lyase, succinic semialdehyde dehydrogenase, sugar-transporting phosphotransferase, ketohydroxyglutarate aldolase, homoserine-O-succinyl transferase, homoserine kinase, homoserine efflux transporter, diaminopimelate decarboxylase, and/or methylglyoxal synthase which is (are) decreased.

In a further aspect, the microorganism of the invention being *Escherichia coli* overexpresses at least one of the genes chosen among *ppc, pck, aceA, galP, asd, thrA, metL, lysC* all *E. coli*; *pycA* from *L. lactis,* and/or has at least one of the genes deleted chosen among *IdhA, adhE, ackA, pta, poxB, focA, pfIB, sad, gabABC, sfcA, mae8, ppc, pykA, pykF, mgsA, sucAB, ptsl, ptsG, pgi,fumABC, aldA, lldD, iclR, metA, thrB, lysA, eda, rhtA, rhtB, rhtC.*

The present invention also encompasses a method of production of 2,4-DHB and/or, by extension, of HMTB and/or HMSeB, in particular a chemical production method of HMTB and/or HMSeB from 2,4-DHB produced according to the invention, comprising the steps of:
- culturing the modified microorganism of the invention in an appropriate culture medium; and
- recovering 2,4-DHB and/or from the culture medium. Said 2,4-DHB can be further purified. Moreover, by adding a methyl-sulfur or a methyl-selenium donor to 2,4-DHB, the method according to the invention allows the production of HMTB and/or HMSeB, respectively.

Product separation and purification is very important factor enormously affecting overall process efficiency and product costs. Methods for product recovery commonly comprise the steps cell separation, as well as product purification, concentration and drying, respectively.

Cell Separation: Ultrafiltration and centrifugation can be used to separate cells from the fermentation medium. Cell separation from fermentation media is often complicated by high medium viscosity. Therefore, we can add additives such as mineral acids or alkali salts, or heating of the culture broth to optimise cell separation.

Product Recovery: A variety of ion-exchange chromatographic methods can be applied for the separation of DHB and/or HMTB and/or HMSeB either before or after biomass removal. They include the use of primary cation exchange resins that facilitate separation of products according to their isoelectric point. Typically, the resin is charged with the solution, and retained product is eluted separately following increase of pH (*e.g.* by adding ammonium hydroxide) in the eluent. Another possibility is the use of ion-exchange chromatography using fixed or simulated moving bed resins. Different chromatographic steps may have to be combined to attain adequate product purity. Those purification methods are more economical compared with a costly crystallization step, also providing additional advantages and flexibility regarding the form of final product.

Product Concentration and Drying: The purification process can also comprise a drying step which may involve any suitable drying means such as a spray granulator, spray dryer, drum dryer, rotary dryer, and tunnel dryer. Concentrated DHB and/or HMTB and/or HMSeB solutions can be obtained by heating fermentation broths under reduced pressure by steam at 130° C. using a multipurpose concentrator or thin film evaporator.

### BRIEF DESCRIPTION OF THE FIGURES

**Figure 1****:** Method of preparation of 2,4-DHB, and by extension, of HMTB and HMSeB, from homoserine comprising a two-step pathway which employs a first step of conversion of the primary amino group of homoserine to a carbonyl group to obtain OHB, and a second step of reduction of the obtained OHB to 2,4-DHB.
**Figure 2****:** Pathway of OHB degradation
**Figure 3****:** *In vivo* DHB consumption and formate production after incubation of wild-type *E.*
*coli* cells and "single aldolase deficient" *E. coli* cells with DHB.
**Figure 4****:** *In vivo* DHB consumption and formate production after incubation of wild-type *E.*
*coli* cells and "multiple aldolase deficient" *E. coli* cells with DHB.
**Figure 5****:** *In vivo* DHB and formate productions during glucose fermentation of "aldolase deficient" *E. coli* "DHB producing" strains.

### EXAMPLES

### 1- Material and methods

### 1.1- Strain and culture conditions

The strains used in the examples are detailed in Table 1.

**Table 1: E. coli strain used in this study**

| **Strain** | **Description** | **Origin** |
|---|---|---|
| *E. coli* substr K.12 | F-lambda-*ilvG-rfb-*50 *rph-*1 | ATCC |
| MG1655 | | n°47046 |
| NEB^{®} 5-alpha | *fhuA2 Δ(argF-lacZ)U169 phoA glnV44 Φ80* | New England |
| competent *E. coli* | *Δ(lacZ)M15 gyrA96 recA1 relA1 endA1 thi-1 hsdR17* | BioLabs |
| BL21 (DE3) | *fhuA2 [lon] ompT gal (λ DE3) [dem] ΔhsdS* | New England |
| | *λ DE3 = λ sBamHlo ΔEcoRI-B* | BioLabs |
| | *int::(lacI::PlacUV5::T7 gene1) i21 Δnin5* | |
| 1160 | *MG1655 ΔthrB::FRT ΔlldD::FRT ΔrhtB::FRT* | This study |
| 1526 | *MG1655 ΔthrB::FRT ΔlldD::FRT ΔrhtB::FRT* | This study |
| | *ΔyjhH::FRT* | |
| 1527 | *MG1655 ΔthrB::FRT ΔlldD::FRT ΔrhtB::FRT* | This study |
| | *ΔyagE::FRT* | |
| 1528 | *MG1655 ΔthrB::FRT ΔlldD::FRT ΔrhtB::FRT* | This study |
| | *ΔyjhH::FRT ΔyagE::FRT* | |

Unless otherwise stated (as may be specified below), the *Escherichia coli* strain K-12 substrain MG1655 (ATCC n°47046) was cultured in a M9 medium containing 0.2% D-xylose as carbon source. When required (as may be specified below), this culture medium also contained 50 mM DHB (racemic mix of L and D DHB ammonium).

### 1.2-Gene Deletions

Deletion of genes coding for pyruvate aldolases (i.e. aldolase according the invention) were carried out in *Escherichia coli* str. K-12 substr. MG1655, using adapted protocols of P1 transduction (Thomason *et al.,* 2007) for *yagE, yjhH, garL, dgoA, yfaU, eda,* and CrispR-Cas9 (Jiang *et al*., 2015) technique was used for *mhpE.*

### 1.3- P1 Transduction

### Preparation of lysate

25 µL of a sterile glucose solution (20% w/v) and 25 µL of sterile CaCl₂ 1M was added to 5 mL of lysogeny broth (LB), and the mix was inoculated with 100 µL of saturated culture of strain from KEIO collection (Baba *et al.,* 2006) harbouring desired deletion, and was let to grow at 37°C with shaking. After 45 min, 100 µL of a P1 stock were added and growth carried for 3 hr or until lysis is complete. Lysed culture was centrifuged at 4000 g, 4°C for 10 min, and the supernatant was filter sterilised. Lysate could be stored for several years at 4°C without loss of efficiency.

### Transduction

1 mL of saturated culture of desired target strain was centrifuged and resuspended with 100 µL of sterile P1 salts solution (10 mM CaCl₂, 5 mM MgSO₄) and 100 µL of P1 lysate carrying the desired mutation. Cells were incubated at 30°C for 30 min without agitation, and a 100 µL of sterile 1 M sodium citrate solution were added to stop the transduction, after what 1 mL of LB was added. Cells were then incubated at 37°C for 1 hr before plating on LB Kanamycine (50 µg/mL).

### Elimination of Kanamycine resistance cassette

Given that kanamycine resistance cassettes in KEIO collection are all flanked on each side with *FRT* sites, the cassette could easily be disrupted by using the Flp enzyme from *Saccharomyces cerevisiae* (Cox, 1983), encoded on pCP20 plasmid (Cherepanov and Wackernagel, 1995). Cells carrying the kanamycine resistance cassette instead of the target gene could be transformed with pCP20 using a protocol described elsewhere (Chung *et al.,* 1989).

Several clones grown resulting of the transduction were picked on the LB Kan plate and used to inoculate 1 mL of LB. When culture reached OD600 of ~0.3, cells were centrifuged and resuspended in TSS solution (tryptone 10 g/L, yeast extract 5 g/L, NaCl 10 g/L, PEG 10 kDa 10 g/L, DMSO 5 g/L and MgCl₂ 20 mM). Cells were left on ice for 15 min before adding 100-150 ng of pCP20 plasmid preparation. After 15 min on ice, a heat shock at 42°C for 1 min was applied to the cells, after what 1 mL of LB was added. Phenotypic expression of beta-lactamase encoded by pCP20 was carried out at 30°C for 1-2 hr with agitation, before plating on LB Ampicillin (100 µg/mL).

Several clones from the LB ampicillin plate were picked and used to inoculated 1 mL of LB, which was then incubated at 42°C for 6-8 hr. A few microliters from the culture were streaked on a LB plate and incubated overnight at 37°C.

A few clones were individually picked from the LB plate and streaked on LB Amp, LB kan and LB plates to counter select the clones that lost the resistance to both ampicillin and kanamycin. Deletions on the clones sensible to those antibiotics were verified by colony PCR using OneTaq^{®} 2X Master Mix with Standard Buffer (BioLabs), following the protocol given by the facturer and using the primers listed in Table 1. Gene deletion was verified by PCR amplification, using primers described in Table 3, in all the strains bearing one of multiple deletion.

**Table 2 - Plasmid used**

| **Plasmid** | **Description** | **Origin** |
|---|---|---|
| pCas | *repA101(Ts) kan Pcas-cas9 ParaB-Red laclq Ptrc*-sgRNA-*pMB1* | (Jiang *et al.,* 2015) |
| pCP20 | *FLP*⁺, λ cl857⁺, λ p_{R} Rept^{ts}, Ap^{R}, Cm^{R} | (Cherepanov and Wackernagel, 1995) |
| pMHPE | pZE13 with genomic region of *mhpE* | This study |
| pMHPEΔ | pZE13 with genomic flanking region of *mhpE* | This study |
| pTargetF | *pMB1 coda* | (Jiang *et al.,* 2015) |
| pTarMHPE | pTargetF expression a gRNA targeting *mhpE* gene | This study |
| pZE13 | colE1 replication origin, Ampicillin resistance, PA1lacO-1 promoter | Expressys |

**Table 3 - Primers used for validation of genes deletion by PCR**

| **Primer name** | **Sequence (5'→3')** | **Use** |
|---|---|---|
| dgoA_seq_F | AGGTCAGCGAATTTCTCTC (SEQ ID NO: 83) | Checking *dgoA* deletion |
| dgoA_seq_R | GATACATCACTTGCCATAAGTC (SEQ ID NO: 84) | Checking *dgoA* deletion |
| eda_seq_F | TCATTCGTGTGAATGGACAG (SEQ ID NO: 85) | Checking *eda* deletion |
| eda_seq_R | TAAGCCGGAAATTGATGGC (SEQ ID NO: 86) | Checking *eda* deletion |
| garL_seq_F | CGCCGAAAGAGATTGTTG (SEQ ID NO: 87) | Checking *garL* deletion |
| garL_seq_R | AGCAATCACGTCAGCAATAG (SEQ ID NO: 88) | Checking *garL* deletion |
| mhpE_cas_F | CGCCAGTAAATCTGCC (SEQ ID NO: 89) | Checking *mhpE* deletion carried out with CrispR |
| mhpE_cas_R | AGGCGGGATGAAGATG (SEQ ID NO: 90) | Checking *mhpE* deletion carried out with CrispR |
| mhpE_seq_F | ATGATATCGAAGCCTCAATC (SEQ ID NO: 91) | Checking *mhpE* deletion |
| mhpE_seq_F | TTATCAGGCCTACAAATTCC (SEQ ID NO: 92) | Checking *mhpE* deletion |
| TM32 | TGTGCTTAACCCAGCAGAG (SEQ ID NO: 130) | Amplification of donor DNA for mhpE deletion |
| TM33 | TACGGGCGAAATGTAGGC (SEQ ID NO: 131) | Amplification of donor DNA for mhpE deletion |
| TM34 | GTCCTAGGTATAATACTAGTCGGTAAAAAACTTTA TATCTGTTTTAGAGCTAGAAATAGC (SEQ ID NO: 93) | Amplification pTarget for deletion of mhpE |
| TM35 | GTCGTTGATCAAAGCTCGCCGCGTTG (SEQ ID NO: 94) | Sequencing verification of pTarget |
| TM36 | GATCTGCGCAACAACAAATAATG (SEQ ID NO: 95) | Forward for *mhpE* deletion |
| TM37 | CATGCCGCTTCTCCTG (SEQ ID NO: 96) | Reverse for *mhpE* deletion |
| TM60 | ACTAGTATTATACCTAGGACTGAG (SEQ ID NO: 97) | Amplification pTarget (reverse) |
| TM61 | GTTATTGTCTCATGAGCGG (SEQ ID NO: 98) | Forward for verification of insert in pZE13 |
| TM62 | CTTTGAGTGAGCTGATACC (SEQ ID NO: 99) | Reverse for verification of insert in pZE13 |
| yagE_seq_F | CCGAACAAGTGTTCACTATG (SEQ ID NO: 100) | Checking *yagE* deletion |
| yagE_seq_R | GATAATCAGCACCTCTTTGC (SEQ ID NO: 101) | Checking *yagE* deletion |
| yfaU_seq_F | TGCAGCTATCGGGTTTATTG (SEQ ID NO: 102) | Checking *yfaU* deletion |
| yfaU_seq_R | TGCTGATATTCCGAACTGTC (SEQ ID NO: 103) | Checking *yfaU* deletion |
| yjhH_seq_F | AGAATTACAAAAAACACGCC (SEQ ID NO: 104) | Checking *yjhH* deletion |
| yjhH_seq_R | CTGAGCAGTAAAATACCGTC (SEQ ID NO: 105) | Checking *yjhH* deletion |

### 1.4- CrispR-Cas9 deletion

The deletion of *mphE* was carried out in CrispR-Cas9, as described elsewhere (Jiang *et al.,* 2015). Firstly, the target strain was transformed as described previously with pCas plasmid carrying Cas9 encoding gene as well as lambda red recombination machinery. pTarMHPE, the plasmid producing the gRNA was constructed based on pTargetF, amplified with primers TM60 and TM34. Correct amplification was verified with the Sanger sequencing services provided by Eurofins Genomics, using the primer TM35.

### Construction of the donor DNA

Reparation fragment was obtained from genomic *Escherichia coli* str. K-12 substr. MG1655. Briefly, *mhpE* region was amplified from genomic DNA preparation of wild type *E. coli* MG1655 with primers TM32 and TM33. The resulting fragment was 1873 bp and was gel purified (EZ-10 Spin Column DNA Gel Extraction Kit by BioBasic) and phosphorylated on its 5' ends using a T4 Polynucleotide Kinase (BioLabs). The vector used was a pZE13 (Expressys) digested with EcoRV (BioLabs), and dephosphorylated with Antarctic phosphatase (BioLabs). The vector and insert were subsequently ligated using a HiT4 DNA Ligase (BioLabs). Quality of the insert was checked by sequencing, using the primers TM61 and TM62.

Resulting circular plasmid (pMHPE) was amplified using primers TM36 and TM37 to linearize and delete the target gene. It was afterward gel purified, phosphorylated and ligated to obtain a pMHPEΔ with genomic region of *mhpE* with missing coding sequence. Finally, the donor DNA for CrispR-Cas9 was amplified from pMHPEΔ with primers TM32 and T33.

### Gene disruption using CrispR-Cas9

The strain carrying pCas plasmid was electroporated with 100 ng of pTarget and 400 ng of donor DNA. Briefly, 50 µL of an overnight culture of target strain were used to inoculate 5 mL LB Kan (50 µg/mL) arabinose (10 mM) and let to grow at 30°C for 3 to 4 hr or until OD600 reached 0.5 -0.7. Then, 2 mL were harvested and placed on ice and pelleted and washed once with 1 mL of ice-cold ddH₂O and once with 1 mL ice-cold 10% glycerol. Cells were then resuspended in 50 µL ice-cold 10% glycerol and transformed 100 ng of pTarget and 400 ng of donor DNA with following conditions: 2 mm cuvette, 2.5 kV, 200Ω, 25 µF, and were immediately resuspended and SOC placed at 30°C overnight. All of the transformation reaction was plated on LB Kanamycine Spectinomycine. Correct genome editing was verified using primers mhpE_cas_F and mhpE_cas_R, before curing the plasmids and verifying the rest of the mutations if needed.

### 1.5- Cloning and characterization of pyruvate aldolase encoding genes

### Cloning methods

The 7 genes encoding pyruvate aldolases were cloned into a pET28 vector (Addgene). Briefly, they were amplified from the start codon to the stop codon with non-flanking 3' ends, as indicated in the Table. 4. With the exception of *eda* and *mhpE,* this base extension allowed to add an Avrll restriction site on the 5' end of the gene and a Mfel site on the 3' end. In case of *eda* and *mhpE,* a different pair of restriction site namely Ndel/Xhol and BamHI/Hindlll were used, respectively. Amplicons were gel purified (BioBasic Gel extraction Kit) before being digested with corresponding restriction enzymes (BioLabs). The pET28 vector was amplified with primers allowing future corresponding ligation, and the amplicons were digested with 1 µL of Dpnl (BioLabs), after what they were cleaned up (BioBasic Gel extraction kit) and digested with the cloning restriction enzymes as well. Digested products were purified and dephosphorylated using the Antarctic Phosphatase (BioLabs). Amplification of the vector allowed addition of Mfel site and also to delete the T7 tag and part of the MCS carried by the vector. For insertion of *mhpE,* pET28 vector was directly used for digestion.

Inserts and vectors were ligated using the HiT4 DNA Ligase (Biolabs), and the result was transformed in NEB^{®} 5-alpha cells (BioLabs). Positive clones were PCR amplified using primers TM89 and TM90 to check correct length of the insert and then validated by sequencing.

**Table 4 - List of primers used to clone pyruvate-aldolase encoding genes in the pET28+ vector**

| **Primer name** | **Sequence (5'→3')** | **Use** |
|---|---|---|
| pet28_Ndel_R | CATGCTAGCCATATGGCTGCC (SEQ ID NO: 106) | pET28 amplification for cloning of *eda* |
| peT28_Xhol_F | ATGCCTCGAGCACCACCACCAC (SEQ ID NO: 107) | pET28 amplification for cloning of *eda* |
| Ndel_eda_F | TCACATATGATGAAAAACTGGAAAACAAGTG (SEQ ID NO: 108) | Amplification of *eda* |
| Xhol_eda_R | TGACCTCGAGTTACAGCTTAGCGCCTTCTAC (SEQ ID NO: 109) | Amplification of *eda* |
| BamH1_mhpE_F | GATCAGGATCCATGAACGGTAAAAAAC (SEQ ID NO: 110) | Amplification of *mhpE* |
| HindIII_mhpE _REV | CAGTCAAGCTTTTATTTGTTGTTGCGC (SEQ ID NO: 111) | Amplification of *mhpE* |
| pET28_AvrlI_R | TCACCTAGGGCCGCTGCTGTGATGATG (SEQ ID NO: 112) | pET28 amplification for insertion of Mfel site |
| pET28_Mfel_F | ATCCAATTGTGAGATCCGGCTGCTAACA (SEQ ID NO: 113) | pET28 amplification for insertion of Mfel site |
| dgoA_AvrlI_F | ATCCCTAGGATGCAGTGGCAAACTAAAC (SEQ ID NO: 114) | Amplification of *dgoA* |
| dgoA_Mfel_R | ATCCAATTGTCATTGCACTGCCTCTC (SEQ ID NO: 115) | Amplification of *dgoA* |
| garL_AvrlI_F | ATCCCTAGGATGAATAACGATGTTTTCCCG (SEQ ID NO: 116) | Amplification of *garL* |
| garL_Mfel_R | ATCCAATTGTTATTTTTTAAAGGTATCAGCCAG (SEQ ID NO: 117) | Amplification of *garL* |
| yagE_AvrlI_F | ATCCCTAGGATGCCGCAGTCCGCGT (SEQ ID NO: 118) | Amplification of *yagE* |
| yagE_Mfel_F | TCACAATTGTCAGCAAAGCTTGAGCTGTTGC (SEQ ID NO: 119) | Amplification of *yagE* |
| yfaU_AvrlI_F | TCACCTAGGATGAACGCATTATTAAGCAATC (SEQ ID NO: 120) | Amplification of *yfaU* |
| yfaU_Mfel_R | TCACATTGTCAATAACTACCTTTTATGCGT (SEQ ID NO: 121) | Amplification of *yfaU* |
| yjhH_AvrlI_F | TCACCTAGGATGAAAAAATTCAGCGGC (SEQ ID NO: 122) | Amplification of *yjhH* |
| yjhH_Mfel_R | TCACAATTGTCAGACTGGTAAAATGCCC (SEQ ID NO: 123) | Amplification of *yjhH* |
| TM89 | CGAGATCTCGATCCC (SEQ ID NO: 124) | PCR verification of the correct insertion in pET28 |
| TM90 | TCCGGATATAGTTCCTCC (SEQ ID NO: 125) | PCR verification of the correct insertion in pET28 |

### Production and purification of the recombinant protein

*E. coli* strain BL21(DE3) were transformed with pET-28a(+) plasmid containing aldolase gene (*yagE, yjhH, yfaU, garL, dgoA* or *eda*)*.* Isolated colonies were then cultivated overnight in a 14 mL culture tube containing 5 mL of LB medium with kanamycin 50 µg/mL at 37 °C and 200 rpm. Cells were transferred in 500 mL Erlenmeyer containing 100 mL of fresh LB medium with kanamycin 50 µg/mL at initial OD600 nm ~0.1. Cultures were incubated at 37 °C and 200 rpm until OD600 nm reached ~0.6. At this stage, 1 mM IPTG was added to the culture medium which was incubated 3 h at 37 °C and 200 rpm. Cells were centrifuged. Pellets were resuspended in lysis buffer (Tris-HCl 50 mM pH 7.5, NaCl 300 mM, imidazole 5 mM) and sonicated for four cycles of 30 sec each at 30% with intermittent 30 sec cooling on ice-ethanol mixture (Fisherbrand). Cell lysates were centrifuged at 14800 rpm for 15 min and supernatant was collected for purification. The HIS-tagged proteins were purified using cobalt TALON His Tag Purification Resin (GE healthcare). Proteins were eluted in 400 µL Tris-HCl 50 mM pH 7.5, NaCl 300 mM, imidazole 200 mM. Purified enzymes were concentrated in 50mM Tris HCl buffer through 10 kDa Amicon Ultra (Millipore). Purification of the protein was verified by SDS-PAGE and protein concentration was measured by spectrophotometry (Epoch², Biotek), considering that 1 mg correspond to 1 OD at 260 nm.

### 1.5- Synthesis of 2-oxo-4-hydroxybutyrate (OHB)

OHB was synthesized from D-homoserine as described previously (Walther *et al.,* 2018). Shortly, 125 mM D-homoserine was incubated with porcine kidney D-amino acid oxidase (1.25 U/mL, Sigma Aldrich) and bovine liver catalase (4400 U/mL, Sigma Aldrich) in 100 mM Tris Buffer at pH 7.8 for 90 min at 37°C. The reaction was purified on an Amicon^{™} Ultra centrifugal filter (cut-off 10 kDa, Millipore) to eliminate the enzymes.

OHB was quantified by mixing 100 µL of purified reaction with 1 mL of 1 M boric acid and 1 M sodium arsenate at pH 6.5. The reaction was incubated at room temperature for 30 min and the absorbance was read at 325 nm. The relation between absorbance and concentration of the ketone was calibrated using pyruvate solutions of known concentrations (Wellner and Lichtenberg, 1971).

### 1.6- Kinetic characterization of pyruvate aldolase on OHB

The aldolase activity was measured by a coupled enzymatic assay with LdhA in excess by oxidation of NADH at 340 nm at 37°C in a microplate reader (Epoch², Biotek). The assay was performed in 250 µL Tris-HCl 50 mM pH 7.5, 5 mM MgCl₂ buffer containing 0.2 mM NADH using varying concentration from 0 to 13 mM OHB. As *E.coli* LDH is not commercially available, this enzyme was produced from BL21(DE3) *E. coli* strain which was transformed with pET-28a(+) plasmid containing *E. coli ldhA* as described above except that the transformant cells were cultivated in 500 mL erlenmeyer containing 100 mL of auto-inducible ZYM-5052 medium (Studier, 2005) with kanamycin 50 µg/mL and cultivated overnight at 37°C and 200 rpm.

### 2- Evidence of OHB degrading pathway

The results are shown in Figure 3.

The data shown that incubation of *E coli* in the presence of 50 mM DHB in the M9 medium with 0.2% D-Xylose led to accumulation of significant amount of formate. Potential importance of this OHB degradation pathway is also highlighted by the fact that more than 70% of the DHB consumed is found as formate (Figure 3).

### 3- Identification of OHB aldolases

As we assumed the existence of OHB aldolase activity with *E. coli,* we search in database (notably Ecocyc) and in literature about potential so-called pyruvate aldolase enzyme. This research led us to identify at least seven of this type of aldolases sharing some versatility that could be able to react with OHB. He *et al.* (2020) expressed in a constitutive manner these aldolases to condense pyruvate and formaldehyde into OHB. While aldolase is catalyzing a reversible reaction, their *in vivo* function might be different. In addition, while the gene encoding these pyruvate aldolases are present in the genome of *E. coli,* it is not obvious that they are either expressed, or even if they are expressed, that they are able to cleave *in vivo* OHB into pyruvate and formaldehyde. Thus, we listed 7 of the pyruvate aldolases (table 5) from previous bibliographic studies (He, H *et al.,* 2020; Clapés *et al.,* 2010), as being able to cleave OHB into pyruvate and formaldehyde in *E. coli* or another microorganism.

**Table 5 - OHB aldolase from Escherichia coli**

| **Aldolase name** | **E.C. number** | **Natural substrate** | **Carbon skeleton length** |
|---|---|---|---|
| YagE | 4.1.2.28 | 2-keto-3-deoxy-D-arabinonate | 5 |
| YjhH | 4.1.2.28 | 2-keto-3-deoxy-D-arabinonate | 5 |
| RhmA (=YfaU) | 4.1.2.53 | 2-keto-3-deoxy-L-rhamnonate | 6 |
| DgoA | 4.1.2.21 | 2-keto-3-deoxy-6-phosphogalactonate | 6 |
| GarL | 4.1.2.20 | 5-dehydro-4-deoxy-D-glucarate | 6 |
| Eda | 4.1.2.14 | 2-keto-3-deoxygluconate 6-phosphate | 6 |
| MhpE | 4.1.3.39 | 4-hydroxy-2-oxopentanoate | 5 |

A transcriptomic analysis during DHB fermentation identified 4 genes encoding aldolases among this list in Table 5 that might be implicated in the degradation of OHB (see Table 6). In particular, *YagE* and *YjhH* encoding aldolase were the most upregulated and this upregulation coincided with maximal formate production during the fermentation.

**Table 6 - Gene expression of OHB aldolase in presence of significant amount of DHB**

| **Aldolase genes** | **Minimal Expression** | **Maximal Expression** |
|---|---|---|
| rhmA | 0.98 | 1.14 |
| garL | 1.06 | 1,32 |
| yagE | 1.82 | 13,33 |
| YjhH | 1.25 | 3.33 |
| yfaU | 0.9 | 1.09 |

We thus carried out need systematic deletion one by one and evaluate effect of these single and combined deletions on the fate of formate production from DHB.

### 4- Kinetic characterization of pyruvate aldolase on OHB

To target most efficient OHB aldolase within the cell, we characterized 5 aldolases. We determine OHB affinity (Km) and catalytic efficiency (Kcat/Km) on *YagE, YjhH, RhmA, DgoA* and *GarL* aldolases.

The results are shown in Table 7. It can be seen that aldolase encoded by yfaU had the highest catalytic efficiency (kcat/Km) but this enzyme displays an affinity ten times superior to that of *YagE* and *YjhH* aldolases. Moreover, the expression of yfaU was not modified during DHB fermentation. These results may suggest that *YagE* and *YjhH* could be best OHB aldolase candidates in our DHB production conditions.

**Table 7 - Kinetic features of pyruvate aldolase on OHB**

| **Enzyme** | **Km (mM)** | **Vmax (µmol/min/mg)** | **k_{cat} (s-1)** | **k_{cat}/Km (s⁻¹.M⁻¹)** |
|---|---|---|---|---|
| **YagE** | 0.35 | 0.630 | 0.040 | 115 |
| **YjhH** | 0.5 | 0.11 | 0.06 | 153 |
| **YfaU** | 4 | 2.1 | 1.56 | 1850 |
| **DgoA** | 2.8 | 0.04 | 0.012 | 4.7 |
| **GarL** | 5.15 | 0.08 | 0.004 | 0.77 |
| **Eda** | > 20 | nd | nd | nd |

### 5- In vivo capacity to degrade OHB by deletion of genes encoding pyruvate aldolase

To evaluate *in vivo* capacity to degrade OHB, and therefore DHB, we performed experiments consisting in incubation of DHB with *E. coli* cells in aerobic conditions in the M9 medium with 0.2% glucose. We measured production of formate in strains that were deleted for one of these aldolase encoding gene.

The results are shown in Figure 3.

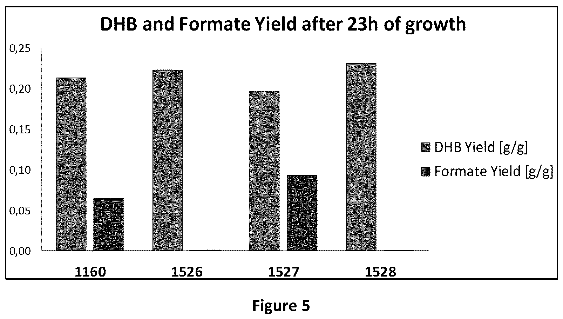

Combined deletions of *yagE* and *yjhH* significantly reduced DHB consumption and formate production (see Figure 4), indicating these enzymes are contributing mostly to the loss of OHB during DHB production by fermentation (see Figure 5). However, deletion of 7 aldolases listed in table 1 led to total loss of OHB production from DHB (see Figure 4).

As these results showed strong reduction of formate, we then evaluate whether the deletion of *yagE* and *yjhH* in the engineered *E. coli* expressing the DHB production pathway improve DHB production during fermentation on glucose. These engineered strains (*i.e.* 1160, 1526, 1527 and 1528 strains, see table 1) harbouring *pZA23* - *thrA_{S345F_}alaC_{A142P Y275D_}mdh_{R81A M85E I12V G179D D86S}* plasmid, wherein mutation in *thrA* alleviates threonine feedback inhibition on ThrA, alaC mutation have been obtained by *in vivo* evolutionary engineered leading to the AlaC^{A142PY275D} variant having a 100-fold higher catalytic activity on homoserine (Bouzon *et al.,* 2017), and mutation in *E.coli mdh* were generated from protein engineering (Frazao *et al.,* 2018) to yield an MDH mutant optimized to reduce OHB.

Inactivation of *yjhH* (strain 1526) leads to a significant improvement in DHB (> 10%) production by increasing glucose yield, while concurrently abolishing formate production (See Figure 5). This effect was less obvious after inactivation of *yagE (strain 1527).* These results are consistent with aldolase biochemical data showing higher catalytic efficiency of YjhH than YagE.

However, combination of *yjhH* and *yagE* deletion (strain 1528) resulted in an even better improvement of DHB production (> 15%). We conclude on synergic effects caused by the double inactivation, probably because lack of one aldolase may be compensated by increase of the other. It should be noted that only DHB yield is a relevant performance indicator because formate can be consumed as a carbon source by cells in DHB production conditions (*i.e.* glucose limited).

### 6- Conclusions

Among all OHB aldolases found in *E. coli,* we clearly identify *YagE* and *YjhH* aldolases are the most efficient to degrade OHB *in vivo* and hence to partially remove OHB away from the DHB pathway. On the contrary, other aldolases like *RhmA* and *GarL* do not have any function on OHB *in vivo,* while they all seem very active in condensing formaldehyde and pyruvate into OHB.

Finally, within the application of DHB production from OHB, we claim that inactivation of *yjhH* and/or *yagE* (both being E.C. 4.1.2.28), is responsible for a significant improvement in DHB production, more especially on glucose yield, and more precisely by abolishing formate production.

### REFERENCES

Altschul, S. F., Gish, W., Miller, W., Myers, E. W. & Lipman D. J., 1990. Basic local alignment search tool. J Mol Biol 215, 403-10.
Altschul, S. F. & Gish, W., 1996. Local alignment statistics. Methods Enzymol 266, 460-80. Altschul, S. F., Madden, T.L., Schäffer, A. A., Zhang, J., Zhang, Z., Miller, W. & Lipman, D.J., 1997. Gapped BLAST and PSI-BLAST: a new generation of protein database search programs. Nucleic Acids Res 25, 3389-402.
Ausubel, M., Brent, R., Kingston, R.E, Moore, D.D., Seidman, J.G., Smith, J.A., & Struhl, K., 1995. Current Protocols in Molecular Biology, Chapter 19. Greene Publishing and Wiley-Interscience, New York.
Baba, T., Ara, T., Hasegawa, M., Takai, Y., Okumura, Y., Baba, M., Datsenko, K.A., Tomita, M., Wanner, B.L., Mori, H., 2006. Construction of Escherichia coli K-12 in-frame, single-gene knockout mutants: the Keio collection. Mol Syst Biol 2.
Bailey, J. E., 1991. Toward a science of Metabolic Engineering. Science 252, 1668-1975. Bouzon, M., Perret, A., Loreau, O., Delmas, V., Perchat, N., Weissenbach, J., Taran, F. & Marlière, P., 2017. A synthetic alternative to canonical one-carbon metabolism. Acs Synthetic Biology. 61520-1533.
Cherepanov, P.P., Wackernagel, W., 1995. Gene disruption in Escherichia coli: TcR and KmR cassettes with the option of Flp-catalyzed excision of the antibiotic-resistance determinant. Gene 158, 9-14.
Chung, C.T., Niemela, S.L., Miller, R.H., 1989. One-step preparation of competent Escherichia coli: transformation and storage of bacterial cells in the same solution. Proc. Natl. Acad. Sci. U.S.A. 86, 2172-2175.
Clapés P., Fessner W.D., Sprenger G.A. & Samland A.K. 2010. Recent progress in stereoselective synthesis with aldolases. Curr Opin Chem Biol. 14, 154-67.
Cox, M.M., 1983. The FLP protein of the yeast 2-eum plasmid: Expression of a eukaryotic genetic recombination system in Escherichia coli. Proc. Natl. Acad. Sci. USA 5.
Dayhoff, M.O., Schwartz, R.M. & Orcutt, B.C., 1978. A model of evolutionary change in proteins. Atlas of Protein Sequence and Structure.
Deck, P., Exner, K. & Buschhaus, B. 2008. Method for the production of D,L-hydroxy-4-alkylthio butyric acid. Edited by B. A G.
Frazão C., Topham C., Malbert Y., Francois J. M. & Walther T. 2018. Rational engineering of a malate dehydrogenase for microbial production of 2,4-dihydroxybutyric acid via homoserine pathway Biochem J 475, 3887-3901.
He H., Höper R., Oodenhöft M., Marlière P. & Bar-Even A. 2020. An optimized methanol assimilation pathway relying on promiscuous formaldehyde-condensing aldolases in E. coli. Metab Eng. 60, 1-13.
Jiang, Y., Chen, B., Duan, C., Sun, B., Yang, J. & Yang, S., 2015. Multigene Editing in the Escherichia coli Genome via the CRISPR-Cas9 System. Appl Environ Microbiol 81, 2506-2514. Needleman, S. B. & Wunsch, C. D., 1970. A general method applicable to the search for similarities in the amino acid sequence of two proteins. J Mol Biol 48, 443-53.
Pearson, W. R & Lipman, D. J., 1988. Improved tools for biological sequence comparison. Proc Natl Acad Sci U S A 85, 2444-8.
Shpaer, E. G., 1997. GeneAssist. Smith-Waterman and other database similarity searches and identification of motifs. Methods Mol Biol 70, 173-87.
Smith, T.F. & Waterman M. S., 1981. Adv. Appl. Math. 2, 482.
Studier FW. (2005) Protein production by auto-induction in high-density shaking cultures. Prot. Exp. Pur. 41, 207-234.
Thomason, L.C., Costantino, N., Court, D.L., 2007. E. coli Genome Manipulation by P1 Transduction. CP Molecular Biology 79.
Walther, T., Calvayrac, F., Malbert, Y., Alkim, C., Dressaire, C., Cordier, H., Francois, J.M., 2018. Construction of a synthetic metabolic pathway for the production of 2,4-dihydroxybutyric acid from homoserine. Metabolic Engineering 45, 237-245.
Wellner, D., Lichtenberg, L.A., 1971. [218a] Assay of amino acid oxidase, in: Methods in Enzymology. Elsevier, pp. 593-596.
Werpy, T. & Petersen, G., 2004. Top Value Added Chemicals from Biomass: Volume I -- Results of Screening for Potential Candidates from Sugars and Synthesis Gas. Pacific Northwest National Laboratory (PNNL) and National Renewable Energy Laboratory (NREL).
Zakataeva N.P., Aleshin V.V., Tokmakova I.L., Troshin P.V. & Livshits V.A., 1999. The novel transmembrane Escherichia coli proteins involved in the amino acid efflux. FEBS Lett. 452, 228-32.

## Claims

1. A method for the preparation of 2,4-dihydroxybutyrate from a carbon source *via* homoserine comprising a two steps pathway:
- a first step of conversion of the primary amino group of homoserine to a carbonyl group to obtain 2-oxo-4-hydroxybutyrate catalysed by an enzyme having homoserine transaminase activity as defined by E.C. 2.6.1.1 or E.C. 2.6.1.42 or E.C. 2.6.1.57 and
- a second step of reduction of the obtained 2-oxo-4-hydroxybutyrate (OHB) to 2,4-dihydroxybutyrate catalysed by an enzyme having OHB reductase activity, the enzyme having OHB reductase activity is lactate dehydrogenase as defined by E.C.1.1.1.27 or E.C.1.1.1.28, or malate dehydrogenase as defined by E.C.1.1.1.37, E.C.1.1.1.82 or E.C.1.1.1.299, and
wherein at least one enzyme having aldolase activity as defined by E.C.4.1.2.28 is inactivated to prevent OHB degradation.

2. The method according to claim 1, wherein the enzyme having aldolase activity is encoded by sequence set forth in SEQ ID NO: 1 or SEQ ID NO: 3, or having a sequence as set forth in SEQ ID NO: 2 or SEQ ID NO: 4 or any sequence encoding an enzyme having aldolase activity and sharing a homology of at least 50% with said sequences.

3. The method according to any preceding claims, wherein the enzyme having homoserine transaminase activity is encoded by gene *ilvE, tyrB, aspC, araT, bcaT, alaC, AROB.*

4. The method according to claim 3, wherein the enzyme having homoserine transaminase activity is:
- encoded by sequence set forth in SEQ ID NO: 5, SEQ ID NO: 7, SEQ ID NO: 9, SEQ ID NO: 11, SEQ ID NO: 13, SEQ ID NO: 15, SEQ ID NO: 126, SEQ ID NO: 128 or any sequence encoding an enzyme having homoserine transaminase activity and sharing a homology of at least 50% with said sequences; or
- corresponds to SEQ ID NO: 6, SEQ ID NO: 8, SEQ ID NO: 10, SEQ ID NO: 12, SEQ ID NO: 14, SEQ ID NO: 16, SEQ ID NO: 127, SEQ ID NO: 129 or any sequence sharing a homology of at least 50% with said sequences.

5. The method of any of preceding claims, wherein the enzyme having OHB reductase is (D)-lactate dehydrogenase from *Escherichia coli,* (L)-lactate dehydrogenase from *Lactococcus lactis,* from *Oryetalagus cuniculus,* from *Geobacillus stearothermophilus,* or from *Bacillus subtilis,* (L)-malate dehydrogenase from *Escherichia coli,* or homologues thereof.

6. The method according to claim 5, wherein the enzyme having OHB reductase is :
- a lactate dehydrogenase comprising at least one mutation in position V17, Q85, E89, I226, or A222 said positions being defined by reference to the L-*Lactis tA* (SEQ. ID NO: 18);
- a malate dehydrogenase comprising at least one mutation in position I12, R81, M85, D86, V93, G179, T211, or M227 said positions being defined by reference to the E.coli Mdh (SEQ ID NO: 20).

7. The method according to claim 5 or 6, wherein the enzyme having OHB reductase activity is:
- encoded by sequence set in forth in SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79 or SEQ ID NO: 81 or any sequence sharing a homology of at least 50% with said sequences; or
- corresponds to SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80 or SEQ ID NO: 82 or any sequence sharing a homology of at least 50% with said sequences.

8. A modified microorganism for the preparation of 2,4-DHB from a carbon source *via* homoserine comprising a two steps pathway comprising :
- a first step of conversion of the primary amino group of homoserine to a carbonyl group to obtain 2-oxo-4-hydroxybutyrate catalysed by an enzyme having homoserine transaminase activity as defined by E.C. 2.6.1.1, E.C. 2.6.1.42 or E.C. 2.6.1.57, and
- a second step of reduction of the obtained 2-oxo-4-hydroxybutyrate to obtain 2,4-dihydroxybutyrate catalysed by an enzyme having OHB reductase activity, the enzyme having OHB reductase activity is lactate dehydrogenase as defined by E.C.1.1.1.27 or E.C.1.1.1.28, or malate dehydrogenase as defined by E.C.1.1.1.37, E.C.1.1.1.82 or E.C.1.1.1.299,
wherein at least one enzyme having aldolase activity as defined by E.C.4.1.2.28 is inactivated to prevent OHB degradation.

9. The modified microorganism according to claim 8, wherein the enzyme having homoserine transaminase activity, the enzyme having OHB reductase activity and the enzyme having aldolase activity are those described in any of claim 2 to 8.

10. The modified microorganism according to claim 8 or 9, wherein the production of homoserine is enhanced compared to the same non-transformed microorganism.

11. The modified microorganism according to any of claims 8 to 10, wherein the activities of the aspartate kinase, aspartate semialdehyde dehydrogenase and/or homoserine dehydrogenase are enhanced.

12. The modified microorganism according to any of claims 8 to 11, wherein the production of 2,4-DHB is enhanced compared to:
- the same non-transformed microorganism; and/or
- a modified microorganism consisting of a first step of conversion of the primary amino group of homoserine to a carbonyl group to obtain 2-oxo-4-hydroxybutyrate, and a second step of reduction of the obtained 2-oxo-4-hydroxybutyrate to obtain 2,4-dihydroxybutyrate.

13. The modified microorganism according to any of claims 8 to 12, being a bacterium, preferentially selected among *Enterobacteriaceae, Clostridiaceae, Bacillaceae, Streptomycetaceae, Si-repi-ococcaceae, Methylobacteriacae,* and *Corynebacteriaceae,* most preferentially *Escherichia coli, Bacillus subtilis, Corynebacterium glutamicum, Clostridium acetobutylicum, Methylobacterium extorquens,* or *Lactococcus lactis,* a yeast preferentially selected among *Saccharomycetaceae, Pichiaceae,* and *Schizosaccharomycetaceae,* most preferentially *Saccharomyces cerevisiae, Schizosaccharomyces pombe, Kluyveromyces lactis, Kluyveromyces marxianus, Pichia jadinii, Pichia stipitis,* or *Pichiapastoris* or a fungus preferentially selected among *Penicillium, Aspergillus, Chrysosporium* or *Trichoderma.*

14. The modified microorganism according to any of claims 8 to 13, wherein the expression of:
- at least one of the enzymatic activities chosen among phosphoenolpyruvate carboxylase, phosphoenolpyruvate carboxykinase, isocitrate lyase, pyruvate carboxylase, and hexose symporter permease is increased; and/or
at least one of the enzymatic activities chosen among lactate dehydrogenase, alcohol dehydrogenase, acetate kinase, phosphate acetyltransferase, pyruvate oxidase, isocitrate lyase, fumarase, 2-oxoglutarate dehydrogenase, pyruvate kinase, malic enzyme, phosphoglucose isomerase, phosphoenolpyruvate carboxylase, phosphoenolpyruvate carboxykinase, pyruvate-formate lyase, succinic semialdehyde dehydrogenase, sugar-transporting phosphotransferase, ketohydroxyglutarate aldolase, homoserine-O-succinyl transferase, homoserine kinase, homoserine efflux transporter, diaminopimelate decarboxylase, and/or methylglyoxal synthase is decreased.

15. The modified microorganism according to claim 14, being *Escherichia coli* overexpressing at least one of the genes chosen among *ppc, pck, aceA, galP, asd, thrA, metL, lysC* all *E. coli; pycA* from *L. lactis,* and/or having at least one of the genes deleted chosen among *IdhA, adhE, ackA, pta, poxB, focA, pflB, sad, gabABC, sfcA, mae8, ppc, pykA, pykF, mgsA, sucAB, ptsl, ptsG, pgi, fumABC,aldA, lldD, iclR, metA, thrB, lysA, eda, rthA, rthB, rthC.*

16. A method of production of 2,4-DHB comprising the steps of:
- culturing the modified microorganism of anyone of claims 8 to 15 in an appropriate culture medium,
- recovering 2,4-DHB from the culture medium.

17. The method according to claim 16, wherein the 2,4-DHB is further purified.
